# EUROPÄISCHE PATENTSCHRIFT

(11) **EP 0 391 396 B1**
(45) Veröffentlichungstag und Bekanntmachung des Hinweises auf die Patenterteilung: **07.06.1995**
(21) Anmeldenummer: 90106469.1
(22) Anmeldetag: 04.04.1990
(51) Int. Cl.: A61B 17/02, A61M 27/00, A61M 25/04

(54) **Auslass- und Instrumentenkanal für die Arthroskopie**
Drainage and instruments canal for arthroscopy
Canal à instruments et de drainage pour l'arthroscopie

(30) Priorität: 07.04.1989 DE 8904371 U
(43) Veröffentlichungstag der Anmeldung: 10.10.1990
(73) Patentinhaber: Dunsch-Herzberg, Renate, D-22880 Wedel (DE); Voss, Gudrun, D-25491 Hetlingen (DE)
(72) Erfinder: Herzberg, Wolfgang, Dr.-med., D-2000 Wedel/Holstein (DE)
(74) Vertreter: Richter, Werdermann & Gerbaulet

(56) Entgegenhaltungen:
- FR-A- 2 343 488
- US-A- 3 539 034
- US-A- 3 938 530
- US-A- 4 069 826
- US-A- 4 608 965
- US-A- 4 699 611

## Beschreibung

Die Erfindung betrifft einen Auslaß- und Instrumentenkanal für die Arthroskopie mit einem Hohlzylinder und einer auf dem Hohlzylinder angeordneten und in Hohlzylinderlängsrichtung verschieblichen Hülse, deren Länge gegenüber der Länge des Hohlzylinders kürzer bemessen ist, der und die Hülse an ihren Enden bündig miteinander verbunden sind und wobei die Hülse mit einer an der Wand des Hohlzylinders feststellbaren, mit dem Hohlzylinder zusammenwirkenden Einrichtung versehen ist und einen bei einem Längsverschieben der Hülse zum Ende des Hohlzylinders hin nach außen spreizbaren und den Hülsenaußendurchmesser korbartig vergrößernden Wandabschnitt mit einer Anzahl von über den Umfang verteilt angeordneten und nebeneinanderliegenden Durchbrechungen aufweist.

Die arthroskopische Operation kann nur im wässrigen Medium vorgenommen werden. Klare Sichtverhältnisse lassen sich nur bei einem stetigen Durchfluß von Flüssigkeit durch das Gelenk erhalten. Läßt sich der Flüssigkeitszufluß über den Optik-Kanal einfach sicherstellen, so ist der ungehinderte Abfluß aus dem Gelenk mit Problemen behaftet:
- Liegt der Ort des Abflusses fern ab vom Ort der Operation - also vom Zufluß -, so ist der Spüleffekt aufgrund der nur laminaren Strömungsverhältnisse am Ort der Operation oft unzureichend; zudem werden freie Knorpel/Meniskuspartikel weit in das Gelenk hineingetragen;
- wird der Ort des Abflusses in die Nähe des Operationsortes gebracht, so ergeben sich häufig räumliche Schwierigkeiten; zudem ist es problematisch, das Abflußrohr aufgrund der engen Verhältnisse daran zu hindern, unbemerkt aus dem Gelenkinnenraum herauszurutschen und sich damit selbst zu verlegen;
- freischwebende Partikel können an die Abflußöffnung herangesogen werden und diese verlegen, da die üblichen Kaliber meist zu klein sind, um die Partikel passieren zu lassen;
- die Entfernung von Partikeln aus dem Gelenk - auch größerer Fragmente - bereitet oftmals Schwierigkeiten; es kann vorkommen, daß ein mit der Zange erfaßter Meniskusteil beim Herausziehen in den Gewebeschichten verlorengeht;
- wird lediglich angestrebt, die Gewebefragmente aus dem Gelenk herauszubefördern, so bleibt ihre Sammlung, um das Gewebe histologisch untersuchen zu können, häufig unberücksichtigt - die Fragmente werden lediglich mit der Flüssigkeit ins Freie gespült.

Bekannt sind diverse stumpfe Ein- und Auslaßkanülen, die zusätzlich mit seitlichen Durchbrechungen versehen sind und im Bereich des Anschlusses einen Absperrhahn tragen. Diese Kanülen sind meist zu dünn und zu lang, so daß sie einerseits gröbere Fragmente nicht passieren lassen, gröbere Zangen ebenfalls nicht aufgrund ihrer Länge für leicht abgewinkelte Instrumente ebenfalls unpassierbar sind. Ferner sind dünne Endlos-Schläuche bekannt, die seitliche Perforationen tragen und fern ab vom Ort der Operation durch das Gelenk hindurchgezogen werden. Ferner sind die sogenannten "Ports" bekannt, die als Führungskanal für die Optik und die Instrumente dienen können. Über eine Gummidichtung wird der Abschluß zum eingeführten Instrument erreicht. Diese Ports sind relativ lang und haben keinen Ankermechanismus, der die Lage im Gelenk sichert.

Durch die FR-A-2 343 488 ist ein Auslaß- und Instrumentenkanal für die Arthroskopie bekannt, der von einem Hohlzylinder und einer auf diesem angeordneten und in Hohlzylinderlängsrichtung verschieblichen Hülse gebildet ist, wobei die Länge der Hülse gegenüber der Länge des Hohlzylinders kürzer bemessen ist. Der Hohlzylinder und die Hülse sind an ihren Enden bündig miteinander verbunden. Die Hülse weist einen bei einem Längsverschieben der Hülse zum Ende des Hohlzylinders hin nach außen spreizbaren und den Hülsenaußendurchmesser korbartig vergrößernden Wandabschnitt mit einer Anzahl von über den Umfang verteilt angeordneten und nebeneinanderliegenden Durchbrechungen auf. Die Arretierung der Hülse auf dem Hohlzylinder erfolgt nur in der Gebrauchs- und NichtgebrauchsStellung, jedoch ist ein stufenweises Verstellen des korbartigen Wandabschnittes zur Ausbildung verschiedener Korbgrößen und ein Arretieren in den verschiedensten Stellungen der Hülse nicht möglich. Außerdem weist dieser Auslaß- und Instrumentenkanal eine große Länge auf, so daß nur begrenzte Einsatzmöglichkeiten gegeben sind.

Der Erfindung liegt die Aufgabe zugrunde, einen luxationssicheren Auslaß- und Instrumentenkanal für die Arthroskopie mit einem möglichst großen Innendurchmesser zu schaffen, der weit in das Gelenk vorschiebbar ist und mit dem die Sicherung der Bergung kleinster Gewebepartikel für später durchzuführende histologische Untersuchungen ermöglicht wird, ohne daß frei gewordene Knorpel/Meniskuspartikelchen in das Gelenk während des Spülvorganges hineingetragen werden. Außerdem soll ein Durchnässen des Umfeldes des Operationsortes durch die Spülflüssigkeit verhindert und eine Fixierung des Auslaß- und Instrumentenkanals ermöglicht werden.

Diese Aufgabe wird durch die im Anspruch 1 gekennzeichneten Merkmale gelöst.

Ein derart erfindungsgemäß ausgebildeter Auslaß- und Instrumentenkanal sichert die Bergung kleinster Gewebepartikel für später durchzuführende histologische Untersuchungen. Der Kanal verhindert ein Durchnässen des Umfeldes des Operationsortes durch die Spülflüssigkeit. Frei gewordene Knorpel/Meniskuspartikelchen werden während des Spülens nicht in das Gelenk hineingetragen. Durch die Arretierbarkeit des Auslaß- und Instrumentenkanals in seiner Gebrauchsstellung wird ein Herausrutschen des Kanals aus dem Gelenkinnenraum vermieden. Des weiteren wird ein Verstopfen der Abflußöffnung durch kleine Partikelchen verhindert.

Der Hohlzylinder des Auslaß- und Instrumentenkanals ist relativ kurz bemessen und weist einen möglichst großen Innendurchmesser auf und ist weit in das Gelenk vorschiebbar, damit die sichere Lage im Gelenk gewährleistet ist. Dadurch, daß der Hohlzylinder in Verbindung mit der auf diesem geführten Hülse eine korbartige Vergrößerung des Außendurchmessers im Endbereich des Auslaß- und Instrumentenkanals ermöglicht, kann der Kanal aus dem Gelenk soweit herausgeführt werden, daß sich das gespreizte Ende an der Gelenkinnenhaut fängt. Die Lage des Hohlzylinders mit seiner Hülse wird hieraufhin dann von außen im Bereich der Haut mit einem Reiter fixiert. Zum Einführen des Auslaß- und Instrumentenkanals wird ein Applikations- bzw. Einführinstrument eingesetzt, welches nach dem Einführen des Auslaß- und Instrumentenkanals entfernt wird, worauf dann ein kurzes T-förmiges Rohrstück auf den Hohlzylinder aufgesetzt wird. Dieses Einführinstrument wird mit dem Auslaß- und Instrumentenkanal so sicher verbunden, daß während des Betriebes ein selbsttätiges Lösen der Verbindung nicht möglich ist. Die waagerechte Achse des Zylinders des T-förmigen Rohrstückes ist mit einem selbstschließenden Ein- und Auslaßmechanismus versehen, wohingegen in dem senkrecht abgehenden Zylinder bzw. Rohr des T-förmigen Rohrstückes ein Absperrhahn bzw. ein Absperrventil vorgesehen ist, mit dem der Abfluß in einen Absaugschlauch regulierbar ist. Nach Beendigung der Operation wird die Spreizung am Ende des Hohlzylinders aufgehoben und der Zylinder mit seiner Hülse aus dem Gelenk herausgezogen.

Der erfindungsgemäß ausgebildete Auslaß- und Instrumentenkanal erbringt folgende Vorteile:
Der korbartig gespreizte Endbereich der Hülse sichert die Lage im Gelenk. Des weiteren ermöglicht der gespreizte Wandabschnitt, das gesamte Instrument nur so knapp als nötig in das Gelenk hineinragen zu lassen, ohne daß dadurch die Gefahr eines Herausgleitens gegeben ist. Seitliche Durchbrechungen im Hohlzylinder im Bereich des korbartig gespreizten Abschnittes der Hülse bieten eine zusätzliche Sicherheit für einen stets offenen Abfluß. Diese Anordnung kann unmittelbar am Ort der Operation angebracht werden, ohne daß es zu einer sich gegenseitig behindernden Anhäufung von Instrumenten kommt.

Der große Innendurchmesser und die Kürze des Hohlzylinders ermöglichen das mühelose Einführen von anderweitigen Instrumenten; größere gefaßte Meniskusfragmente können ohne Schwierigkeiten über diesen Kanal entfernt werden. Auch an der Spitze leicht gekrümmte Instrumente können eingesetzt werden. Der im Nebenschluß angebrachte Absaugschlauch ist über einen Hahn regulierbar. Dadurch ist die Bergung kleinerer Partikel für die spätere histologische Untersuchung gesichert und es wird verhindert, daß sich Spülflüssigkeit über anderweitige Leckagen des Gelenkes einen Weg ins Freie sucht und dadurch das Umfeld der Operation durchnäßt. Der Absaugschlauch kann mit einem Auffangbehälter verbunden sein, um abgelöste Partikel von der Spülflüssigkeit trennen zu können.

Vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen
Fig. 1 in einer schaubildlichen Ansicht einen aus einem Hohlzylinder mit aufgesetzter, verschiebbarer Hülse bestehenden Auslaß- und Instrumentenkanal,
Fig. 2 in einer schaubildlichen Ansicht den Auslaß- und Instrumentenkanal in einer um 180° in der Längsachse gedrehten Stellung,
Fig. 3 in einer schaubildlichen Ansicht den Auslaß- und Instrumentenkanal mit einendseitig ausgespreiztem Wandabschnitt,
Fig. 4 in einem senkrechten Längsschnitt den Hohlzylinder mit aufgeschobener Hülse und einer die Hülse auf dem Hohlzylinder feststellbaren Einrichtung aus einem in ein Schuppenprofil eingreifenden Einrastnocken,
Fig. 5 in einer schaubildlichen Ansicht einen Reiter zum Arretieren des Auslaß- und Instrumentenkanals in einem außerhalb der Haut liegenden Bereich,
Fig. 6 eine Vorderansicht des Reiters gem.Fig. 4,
Fig.7 in einer schaubildlichen Ansicht den Auslaß- und Instrumentenkanal mit einem die Hülse in ihrer Betriebsstellung arretierenden Reiter,
Fig.8 bis 11 in schaubildlichen Ansichten den im Bereich eines Gelenkes für eine arthroskopische Operation angeordneten Auslaß- und Instrumentenkanal in verschiedenen Stellungen und
Fig.12 bis 16 in Seitenansichten den im Bereich eines Gelenkes für eine arthroskopische Operation angeordneten Auslaß- und Instrumentenkanal in verschiedenen Stellungen.

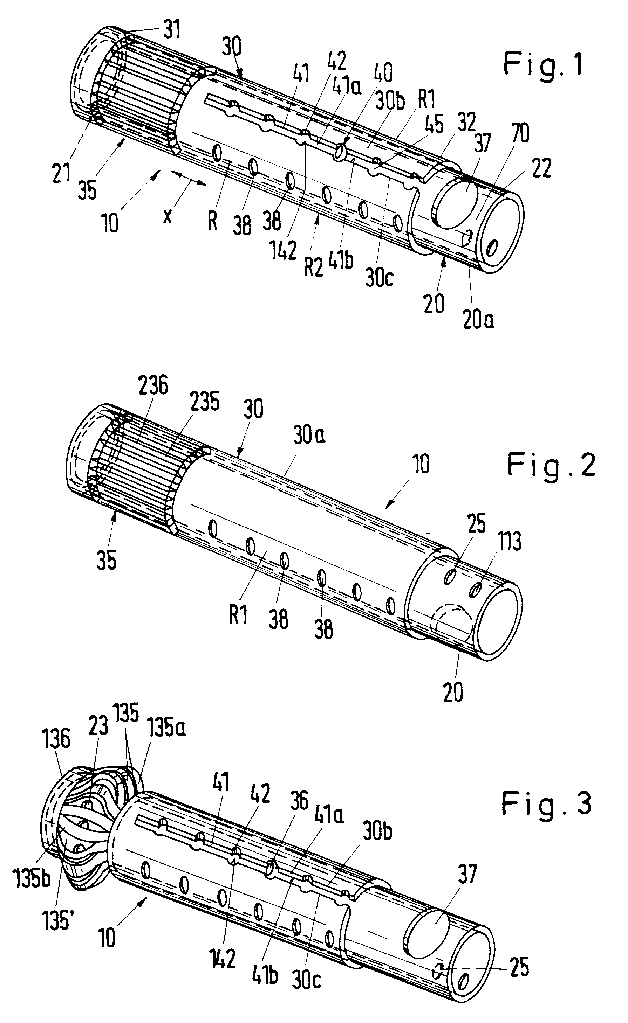

Der in Fig. 1 bis 3 dargestellte und mit 10 bezeichnete Auslaß- und Instrumentenkanal stellt ein rohrförmiges Instrument mit kanülenartigem Charakter dar.

Dieser Auslaß- und Instrumentenkanal 10 besteht aus einem Hohlzylinder 20 und einer auf diesem in Hohlzylinderlängsrichtung verschieblichen Hülse 30, deren Länge gegenüber der Länge des Hohlzylinders 20 kürzer bemessen ist. Der Hohlzylinder soll möglichst kurz bemessen sein, z.B. etwa 5 cm, und mit einem möglichst großen Innendurchmesser versehen sein, z.B. von etwa 8 mm. Die beiden Enden des Hohlzylinders 20 sind mit 21,22, die Hohlzylinderwand mit 20a und die Hülsenwand mit 30a bezeichnet. Im Bereich ihrer Enden 21,31 sind der Hohlzylinder 20 und die Hülse 30 bündig miteinander verbunden und in diesen Endbereichen verschlossen ausgebildet.

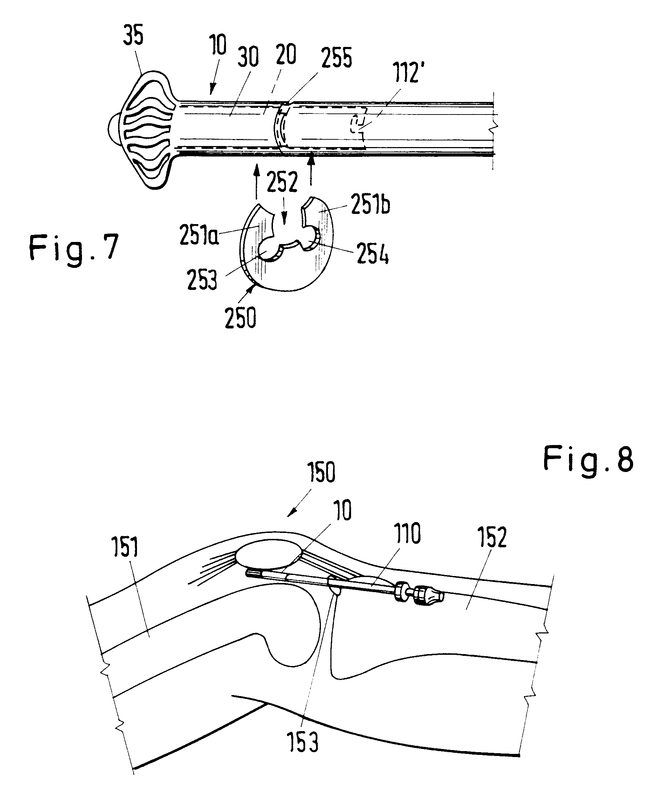

Der im Bereich des Endes 31 der Hülse 30 liegende Wandabschnitt 35 der Hülse ist so ausgebildet, daß dieser Wandabschnitt 35 bei einem Verschieben der Hülse 30 in Pfeilrichtung X sich korbartig nach außen unter Ausbildung einer Anzahl von über den Umfang verteilt angeordneten und parallel zueinander verlaufenden schlitzförmigen Durchbrechungen 135' spreizt, so daß ein Auslaß- und Instrumentenkanal 10 erhalten wird, der in seinem Endbereich einen den Hülsenaußendurchmesser vergrößernden Wandabschnitt 35 aufweist (Fig.7).

Im Bereich des spreizbaren Wandabschnittes 35 weist der Hohlzylinder 20 eine Anzahl von über den Hohlzylinderumfang verteilt angeordneten Durchbrechungen 23 auf. Die Wandabschnittsspreizung der Hülse 30 wird durch eine nachstehend noch näher beschriebene Arretierung der auf dem Hohlzylinder 20 verschobenen Hülse 30 aufrechterhalten (Fig.3).Hierzu ist die Hülse 30 mit einer die Hülse an der Wand 20a des Hohlzylinders 20 feststellbaren, mit dem Hohlzylinder 20 zusammenwirkenden Einrichtung 40 versehen, die nachstehend näher beschrieben wird. Der Hohlzylinder 20 weist an seinem Ende 22 einen an der Hohlzylinderwand 20a befestigten Führungsnocken 25 für die Hülse 30 auf (Fig.2).

Der nach außen spreizbare und korbartig erweiterbare Wandabschnitt 35 der Hülse 30 besteht aus einer Anzahl von über den Hülsenumfang verteilt angeordneten und in Hülsenlängsrichtung verlaufenden lamellen- bzw. stegartigen Formkörpern 135, die mit ihren Enden 135a an dem Körper der Hülse 30 und mit ihren anderen Enden 135b an einem Ringkörper 136 befestigt sind, der mit dem Hohlzylinder 20 fest verbunden ist. Der Ringkörper 136 bildet mit dem Körper der Hülse 30 über die lamellenartigen Formkörper 135 eine Baueinheit (Fig.3).

Der spreizbare Wandabschnitt 35 der Hülse 30 kann jedoch auch von einer Anzahl von über den Umfang verteilt angeordneten und in Hülsenlängsrichtung verlaufenden, in der Wand 30a der Hülse 30 ausgebildeten Schlitzen 236 und den jeweils zwischen den Schlitzen 236 verbliebenen streifenförmigen Wandabschnitten 235 gebildet sein, wobei diese Wandabschnitte 235 den lamellenartigen Formkörpern 135 entsprechen (Fig.2).

Die lamellenartigen Formkörper 135 und die streifenförmigen Wandabschnitte 235 der Hülse 30 sind dünnwandig ausgebildet; sie bestehen aus einem federnd-elastischen Werkstoff, so daß bei einem Verschieben der Hülse 30 in Pfeilrichtung X diese lamellenartigen Formkörper 135 bzw. streifenförmigen Wandabschnitte 235 nach außen gespreizt werden (Fig. 1 und 3).

Die Hülse 30 ist auf dem Hohlzylinder 20 bei nach außen erweitertem Wandabschnitt 35 mittels der Einrichtung 40 feststellbar. Diese Einrichtung 40 zum Feststellen der Hülse 30 auf dem Hohlzylinder 20 besteht nach einer ersten Ausführung der Erfindung aus einem in Hülsenlängsrichtung verlaufenden und in das dem spreizbaren Wandabschnitt 35 der Hülse 30 abgekehrten Ende 32 auslaufenden und in der Wand der Hülse 30 ausgebildeten Schlitz 41. In den sich gegenüberliegenden Längsrändern 41a,41b der den Schlitz 41 begrenzenden Wandabschnitte 30b,30c der Hülse 30 sind in gleichen oder ungleichmäßigen Abständen teilkreisförmige Ausnehmungen 42,142 ausgebildet, wobei die Ausnehmungen 42 im Wandabschnitt 30b den Ausnehmungen 142 im Wandabschnitt 30c gegenüberliegend sind, so daß je zwei sich gegenüberliegende Ausnehmungen 42,142 sich zu einer etwa kreisförmigen Ausnehmung 45 ergänzen. Um die Hülse 30 auf dem Hohlzylinder 20 arretieren zu können, ist der Hohlzylinder 20 mit einem im Bereich des Schlitzes 41 der Hülse 30 liegenden Feststellnocken 36 versehen, dessen Formgebung und Durchmesser in etwa den kreisförmigen Ausnehmungen 45 entsprechen, die von den sich gegenüberliegenden Ausnehmungen 42,142 gebildet werden. Die Breite des Schlitzes 41 in der Hülse 30 ist so bemessen, daß beim Einschieben des Hohlzylinders 20 in den Innenraum der Hülse 30 der Feststellnocken 36 in den Schlitz 41 bei gleichzeitiger geringfügiger Spreizung der sich gegenüberliegenden und den Schlitz 41 begrenzenden Wandabschnitte 30b,30c der Hülse 30 einführbar ist, so daß bei Erreichen einer Ausnehmung 45 der Feststellnocken 36 in dieser Ausnehmung 45 gehalten ist und dadurch die Hülse 30 auf dem Hohlzylinder 20 unverrückbar befestigt ist (Fig.3). Der Schlitz 41 in der Wand 30a der Hülse 30 ist in Hülsenlängsrichtung verlaufend ausgebildet und mündet in der Hülsenöffnung im Bereich des Endes 32 der Hülse 30.

Anstelle einer derart ausgebildeten Feststelleinrichtung 40 kann das Feststellen der Hülse 30 an dem Hohlzylinder 20 auch mittels geeigneter, andersartig ausgebildeter Einrichtungen erfolgen, z.B. vermittels Rasteinrichtungen u.dgl.. Durch die Anzahl der im Bereich des Schlitzes 41 der Hülse 30 ausgebildeten Ausnehmungen 45 durch die sich gegenüberliegenden Ausnehmungen 42,142 ist die Hülse 30 auf dem Hohlzylinder 20 in jeder Stellung arretierbar, so daß die Größe des Umfanges der korbartigen Enderweiterung der Hülse 30 den jeweiligen Erfordernissen entsprechend variiert werden kann. Der Feststellnocken 36 kann auch eindrückbar ausgebildet sein, um ein Verschieben der Hülse 30 in Pfeilrichtung X1 bei eingedrückter Stellung des Feststellnockens 36 zu ermöglichen. Bei dieser Ausführungsform ist dann der Feststellnocken 36 selbsttätig rückstellbar ausgebildet, was durch eine entsprechende Werkstoffauswahl erfolgen kann.

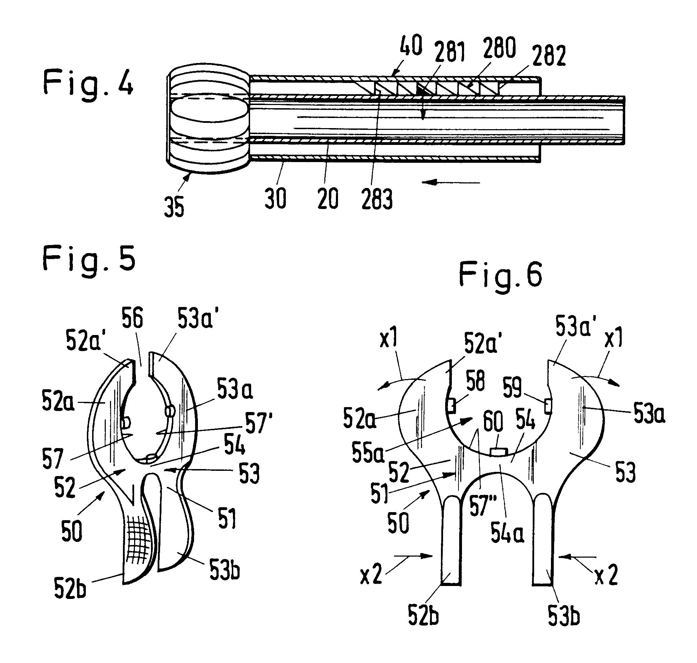

Eine weitere Ausführungsform einer Feststelleinrichtung 40 für die Hülse 30 auf dem Hohlzylinder 20 ist in Fig.4 dargestellt. Hiernach besteht die Einrichtung 40 aus einem auf der Innenwand der Hülse 30 angeordneten schuppenartigen Profilkörper 280 und aus einem Einrastnocken 281 an der Außenwand des Hohlzylinders 20, wobei das Schuppenprofil 282 des Profilkörpers 280 Anschlagkanten 283 für den Einrastnocken 281 aufweist, die dem rückwärtigen Ende der Hülse 30 zugekehrt sind. Der Einrastnocken 281 für ein Lösen der Arretierung kann in die Wand des Hohlzylinders 20 einschwenkbar oder einziehbar sein.

Das Feststellen der Hülse 30 auf dem Hohlzylinder 20 und das Einführen des Auslaß- und Instrumentenkanals 10 in den Bereich eines Gelenkes für eine arthroskopische Operation gemäß Fig. 8 bis 16 erfolgt mittels eines Applikations- bzw. Einführinstruments 110, das beim Aufsetzen auf den Hohlzylinder 20 ein Vorschieben der Hülse 30 bewirkt, um den endseitigen Wandabschnitt des Auslaß- und Instrumentenkanals 10 korbartig zu spreizen bzw. zu erweitern und in der gespreizten Stellung zu halten,was gemäß Fig.7 vermittels eines auf den Hohlzylinder 20 aufgesetzten Reiters 250 erfolgt, worauf nachstehend noch näher eingegangen wird.

Der Hohlzylinder 20 weist im Bereich seines Endes 22 noch eine weitere größere Öffnung 37 auf (Fig.1).

Der Hohlzylinder 20 und die Hülse 30 bestehen aus geeigneten Werkstoffen, insbesondere Stahl oder anderen geeigneten und für die Herstellung von Operationsbestecken und -instrumenten geeigneten Werkstoffen. Jedoch auch andere geeignete Werkstoffe können zur Anwendung gelangen.

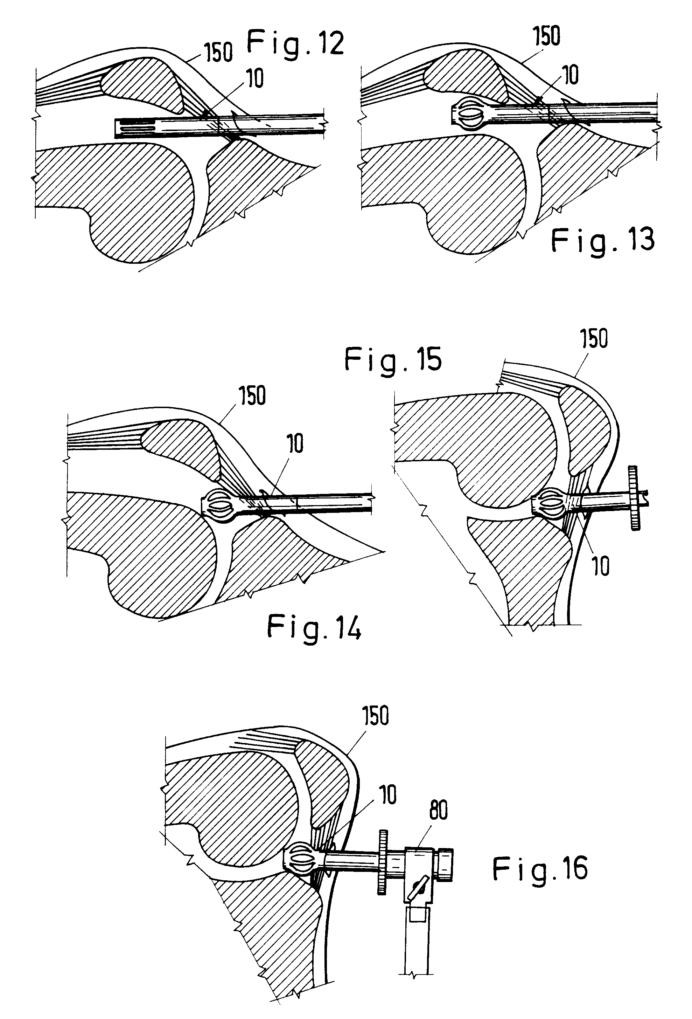

Um den Auslaß- und Instrumentenkanal 10 in der Gebrauchsstellung in seiner jeweils erforderlichen Lage sichern zu können, ist ein an dem Auslaß- und Instrumentenkanal 10 festsetzbarer Reiter 50 vorgesehen. Dieser Reiter ist auf der den Hohlzylinder 20 umgreifenden Hülse arretierbar gehalten und umgreift die Wand 30a der Hülse 30 (Fig.11).

Dieser Reiter 50 besteht gemäß Fig.5 und 6 aus einem H-förmigen Formkörper 51, dessen seitlichen Längsschenkel mit 52,53 und der die Längsschenkel mittig verbindende Steg mit 54 bezeichnet sind. Die oberhalb des die beiden Längsschenkel 52,53 verbindenden Steges liegenden Schenkelabschnitte 52a,53a sind teilkreisförmig ausgebildet und ergänzen sich zu einem offenen Ring 55, wobei die beiden freien Enden 52a',53a' der Schenkelabschnitte 52a,53a sich gegenüberliegend angeordnet sind (Fig.6). Der Zwischenraum zwischen diesen beiden Enden 52a',53a' der beiden Schenkelabschnitte 52a,53a ist bei 56 angedeutet. Der Abstand der beiden Enden 52a',53a' der Schenkelabschnitte 52a,53a ist so bemessen, daß bei einem gleichzeitigen leichten Spreizen der Schenkelabschnitte 52a,53a in Pfeilrichtung X1 der Reiter 50 auf die Hülse 30 aufgesetzt werden kann, so daß die beiden Schenkelabschnitte 52a,53a die Außenwandfläche der Hülse 30 abschnittsweise umgreifen. Die von den beiden Schenkelabschnitten 52a,53a begrenzte und umschlossene Kreisfläche 55a weist in etwa einen Durchmesser auf, der dem Außendurchmesser der Hülse 30 entspricht.

Das leichte, seitliche Auseinanderspreizen der Schenkelabschnitte 52a,53a des Reiters 50 in Pfeilrichtung X1 wird vermittels der unteren Schenkelabschnitte 52b,53b der Längsschenkel 52,53 des Reiters 50 erreicht, die handhabenartig ausgebildet sind (Fig.5). Der die beiden Längsschenkel 52,53 des Reiters 50 verbindende Steg 54 ist in seiner Länge kurz bemessen und dünnwandig ausgebildet und besteht vorzugsweise aus federnd-elastischen Werkstoffen, so daß bei einem Zusammendrücken der unteren Schenkelabschnitte 52b,53b in Pfeilrichtung X2 die beiden oberen Schenkelabschnitte 52a,53a in Pfeilrichtung X1 gespreizt werden. Anstelle einer federnd-elastischen Ausgestaltung des Steges 54 des Reiters 50 kann diese Elastizität und ein gleichzeitig damit verbundenes Rückstellvermögen auch durch die Wahl eines geeigneten Werkstoffes erreicht werden. So besteht z.B. die Möglichkeit, den Reiter 50 aus Kunststoff oder Stahl herzustellen, wobei dann der Steg 54 in seinen Abmessungen so gehalten ist, daß ein Abbiegen des Steges 54 in dem mit 54a gekennzeichneten Bereich möglich ist (Fig.6).

Der Reiter 50 weist zur Lagensicherung auf der Hülse 30 an den einanderzugekehrten Innenkanten 57,57' der beiden oberen Schenkelabschnitte 52a,53a zwei sich gegenüber-liegende Eingriffnocken 58,59 und einen weiteren Eingriffnocken 60 auf, der zwischen den Eingriffnocken 58,59 an der im Bereich des Steges 54 des Formkörpers 51 liegenden Innenkante 57'' befestigt ist, so daß die beiden Eingriffnocken 58,59 sich gegenüberliegen und der Nocken 60 mittig und unterhalb dieser beiden Eingriffnocken 58,59 liegt (Fig.6).

Für das Festsetzen des Reiters 50 auf der Hülse 30 weist die Hülse, die auf dem Hohlzylinder 20 sitzt, in ihrer Wand 30a drei in Hülsenlängerichtung verlaufende Reihen R,R1,R2 von hintereinanderliegend angeordneten Durchbrechungen 38 auf, wobei die beiden Reihen R,R1 sich gegenüberliegend sind, wohingegen die Reihe R2 mittig und unterhalb der beiden Reihen R,R1 liegend ist (Fig.1 und 2). Jede Reihe R,R1,R2 weist eine Anzahl von im Abstand voneinander liegenden Durchbrechungen 38 auf, wobei gleiche oder auch ungleiche Abstände zwischen den Durchbrechungen 38 gegeben sein können. Die Anordnung dieser drei Reihen R,R1,R2 mit den Durchbrechungen 38 steht in Übereinstimmung mit der Anordnung der Eingriffnocken 58,59,60 des Reiters 50, so daß bei einem Aufsetzen des Reiters 50 auf die Hülse 30 die drei Eingriffnocken 58,59,60 in jeweils drei, in einer senkrechten, zur Hülsenlängsachse verlaufenden Ebene liegende Ausnehmungen 45 eingreifen. In den Fig. 1 bis 3 sind die Reihen R und R1 mit ihren Durchbrechungen 38 sichtbar dargestellt, wohingegen die Reihe R2 nicht sichtbar ist.

Die beiden oberen Schenkelabschnitte 52a,53a des Formkörpers 51 des Reiters 50 weisen vorzugsweise eine dem Umfang der Hülse 30 entsprechende Formgebung auf, zumal sowohl der Hohlzylinder 20 als auch die Hülse 30 andere Querschnittsflächenformen als Kreisflächen aufweisen können.

Das dem spreizbaren Wandabschnitt 35 der Hülse 30 abgekehrte Ende des Hohlzylinders 20 ist als Anschlußstutzen 70 für weitere Einrichtungen, wie z.B. eines Applikationsinstrumentes (Fig.8) ausgebildet.

Um die Hülse 30 auf dem Hohlzylinder 20 in der Hülsenstellung zu arretieren, bei der das Hülsenende korbartig erweitert ist (Fig. 7), weist der Hohlzylinder 20 in seiner Außenwandfläche eine Ringnut 255 zur Aufnahme der Schenkel 251a,251b eines auf den Hohlzylinder 20 aufgesetzten Reiters 250 auf, der klemmend an dem Hohlzylinder 20 gehalten ist.

Der Reiter 250 besteht gemäß Fig. 7 aus einem etwa U-förmigen Formkörper 251 mit den Hohlzylinder 20 umgreifenden, federnd-elastischen Schenkeln 251a,251b, die innenwandseitig ein dem Hohlzylinderprofil entsprechendes Profil aufweisen. Der Formkörper 251 ist mit zwei mit der Hohlzylinderaufnahmeöffnung 252 des Reiters 250 verbundene Öffnungen 253,254 zur Aufnahme von Schläuchen, Kabeln od.dgl. versehen. Der Reiter 250 ist klemmend auf dem Hohlzylinder 20 zur Verhinderung eines Zurückgleitens der Hülse 30 aus der Betriebsstellung mit korbartig erweitertem Endumfang der Hülse 30 in die Ausgangsstellung gehalten.

Die Ringnut 255 an dem Hohlzylinder 20 ist so angeordnet, daß nach einem Vorschieben der Hülse 30 vermittels eines Überwurfrohres und nach erfolgter Spreizung bzw. Erweiterung des Hülsenendes die Hülse 30 auf dem Hohlzylinder 20 arretiert ist. Durch Lösen des Reiters 250 von dem Hohlzylinder 20 ist die Hülse 30 entriegelt und wird in ihre Ausgangsstellung (Fig. 1 und 2) zurückbewegt. Durch diese Rückbewegung der Hülse 30 wird die Spreizung am Hülsenende aufgehoben. Das Rückführen der Hülse 30 auf dem Hohlzylinder 20 kann dabei von einer Druck- oder Zugfeder oder einem anderen federnden Element bewirkt werden.

Die Handhabung des Auslaß- und Instrumentenkanals 10 wird anhand der Fig. 8 bis 16 nachstehend näher beschrieben.

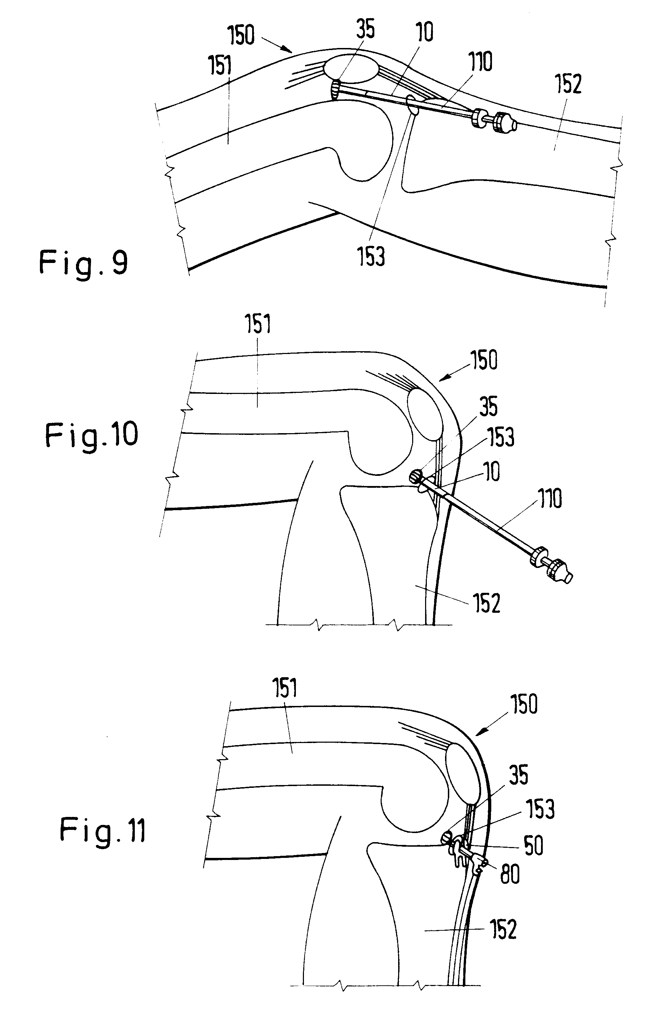

Mit 150 ist ein Gelenk und mit 151,152 die beiden das Gelenk bildenden Knochen bezeichnet. 153 ist die in der das Gelenk umgebenden Haut angebrachte Öffnung zum Einführen des Auslaß- und Instrumentenkanals 10. Durch diese Öffnung 153 in der Haut wird der Auslaß- und Instrumentenkanal 10 bis in den Gelenkbereich eingeschoben, und zwar unter Verwendung des Führungs- bzw. Applikations- bzw. Einführinstrumentes 110, das gleichzeitig zur Führung des Kanals 10 dient (Fig.8). Dadurch, daß der Hohlzylinder 20 kurz bemessen ist, besteht die Möglichkeit, den Hohlzylinder mit seiner Hülse 30 weit in das Gelenk vorzuschieben, damit eine sichere Lage im Gelenk gewährleistet ist. Durch entsprechende Betätigung des Applikationsinstrumentes 110 wird am freien Ende des Hohlzylinders 20 durch Verschieben der Hülse 30 der Wandabschnitt 35 gespreizt und damit eine wesentliche Zunahme des Außendurchmessers erreicht (Fig.9,14 bis 16). Hieraufhin wird der Hohlzylinder 20 bei nach außen gespreiztem Wandabschnitt 35 so weit aus dem Gelenk herausgeführt, bis sich der gespreizte Abschnitt an der Gelenkinnenhaut fängt (Fig.10). Diese Lage des Hohlzylinders 20 wird nun von außen im Niveau der Haut mit dem Reiter 50 fixiert. Das Führungs- bzw. Applikationsinstrument 110 wird daraufhin entfernt und dann auf den Hohlzylinder 20 ein T-förmiges Rohrstück 80 für den Anschluß eines Absaugschlauches aufgesetzt (Fig.11). Nach Beendigung der Operation wird die Spreizung aufgehoben und der Hohlzylinder 20 aus dem Gelenk herausgezogen.

Bei der in Fig. 12 bis 16 gezeigten Handhabung des Auslaß- und Instrumentenkanals 10 ist kein Reiter 250 vorgesehen. Der Auslaß- und Instrumentenkanal ist auch ohne Reiter 250 einsetzbar; in diesem Fall erfolgt die Arretierung der Hülse 30 auf dem Hohlzylinder 20 bei gespreiztem Hülsenende mit anderen, technischen, voranstehend beschriebenen Einrichtungen. Für die Rückführung des Auslaß- und Instrumentenkanals 10 wird die Hülsenverriegelung gelöst und durch Rückführen des Überwurfrohres 114 wird das Hülsenende aus der gespreizten Stellung (Fig.3) in die Ausgangsstellung zurückgeführt (Fig.1).

Im Betriebszustand weist das gespreizte Ende der Hülse 30 in etwa die Form einer Kugel oder eines Ellipsoids auf (Fig.3).

## Patentansprüche

1. Auslaß- und Instrumentenkanal für die Arthroskopie mit einem Hohlzylinder (20) und einer auf dem Hohlzylinder (20) angeordneten und in Hohlzylinderlängsrichtung verschieblichen Hülse (30), deren Länge gegenüber der Länge des Hohlzylinders (20) kürzer bemessen ist, wobei die Hülse (30) mit einer an der Wand (20a) des Hohlzylinders (20) feststellbaren, mit dem Hohlzylinder (20) zusammenwirkenden Einrichtung (40) versehen ist und einen bei einem Längsverschieben der Hülse (30) zum Ende (21) des Hohlzylinders (20) hin nach außen spreizbaren und den hülsenaußendurchmesser korbartig vergrößernden Wandabschnitt (35) mit einer Anzahl von über den Umfang verteilt angeordneten und nebeneinanderliegenden Durchbrechungen (135) aufweist, dadurch gekennzeichnet, daß die Einrichtung (40) zum Feststellen der Hülse (30) auf dem Hohlzylinder (20) aus einem in Hülsenlängsrichtung verlaufenden an der Hülse (30) ausgebildeten Eingriffprofil (41, 45; 280) und aus einem im Bereich des Eingriffprofils (41, 45; 280) der Hülse (30) auf der Außenwand (20a) des Holzylinders (20) angeordneten Feststell- bzw. Einrastnocken (36, 281) besteht, und daß zur Lagensicherung des Auslaß- und Instrumentenkanals (10) in seiner Gebrauchsstellung die den Hohlzylinder (20) umgreifende Hülse (30) auf ihrer Wand (30a) einen an dieser arretierbaren Reiter (50, 250) trägt.

2. Auslaß- und Instrumentenkanal nach Anspsruch 1, dadurch gekennzeichnet, daß die Einrichtung (40) zum Feststellen der Hülse (30) auf dem Hohlzylinder (20) aus einem in Hülsenlängsrichtung verlaufenden und in das dem spreizbaren Wandabschnitt (35) der Hülse (30) abgekehrten Ende (32) auslaufenden und in der Hülsenwand ausgebildeten Schlitz (41) mit einer Anzahl von in gleichen oder ungleichmäßigen Abständen angeordneten teilkreisförmigen Ausnehmungen (42, 142) in den sich gegenüberliegenden Längsrändern (41a, 41b) der den Schlitz (41) begrenzenden Wandabschnitte (30b, 30c) der Hülse (30) besteht, wobei sich je zwei gegenüberliegende Ausnehmungen (42, 142) zu einer etwa kreisförmigen Ausnehmung (45) ergänzen, und daß auf der Außenwand (20a) des Hohlzylinders (20) im Bereich des Schlitzes (41) der Hülse (30) ein in etwa dem Durchmesser der Ausnehmung (45) entsprechend bemessener Feststellnocken (36) angeordnet ist und daß zur Lagensicherung des Auslaß- und Instrumentenkanals (10) in seiner Gebrauchsstellung die den Hohlzylinder (20) umgreifende Hülse (30) auf ihrer Wand (30a) einen an dieser arretierbaren Reiter (50,250) trägt.

3. Auslauf- und Instrumentenkanal nach Anspruch 1, dadurch gekennzeichnet, daß die Einrichtung (40) zum Feststellen der Hülse (30) auf dem Hohlzylinder (20) aus einem auf der Innenwand der Hülse (30) angeordneten, schuppenartigenProfilkörper (280) und aus einem Einrastnocken (281) an der Außenwand des Hohlzylinders (20) besteht, wobei das Schuppenprofil (282) des Profilkörpers (280) Anschlagkanten (283) für den Einrastnocken (281) aufweist, die dem rückwärtigen Ende der Hülse (30) zugekehrt sind, und daß der Einrastnocken (281) für ein Lösender Arretierung in die Wand des Hohlzylinders (20) einschwenkbar ist.

4. Auslaß- und Instumentenkanal nach einem der Ansprüche 1 bis 3, dadurch gekennzeichnet, daß zur Aufnahme des Reiters (50) die Hülse (30) des Hohlzylinders (20) in der Wand (30a) drei in Hülsenlängsrichtung verlaufende Reihen (R, R1, R2) hintereinanderliegend angeordneter Durchbrechnungen (38) aufweist, wobei von diesen drei Reihen (R, R1, R2) mit den Durchbrechungen (38) zwei Reihen (R, R1) seitlich und die dritte Reihe (R2) zwischen den beiden Reihen (R, R1) und unterhalb diesen in der Wand (30a) der Hülse (30) ausgebildet sind.

5. Auslaß- und Instrumentenkanal nach einem der Ansprüche 1 bis 4, dadurch gekennzeichnet, daß der Reiter (50) aus einem H-förmigen Formkörper (51) mit oberhalb des die beiden Längsschenkel (52, 53) verbindenden Steges (54) liegenden, sich zu einem offenen Ring (55) ergänzenden, teilkreisförmigen Schenkelabschnitten (52a, 53a) und mit als Handhaben ausgebildeten unteren Schenkelabschnitten (52b, 53b) besteht, wobei an den einander zugekehrten Innenkanten (57, 57') der beiden oberen Schenkelabschnitte (52a, 53a) zwei sich gegenüberliegende Eingriffnocken (58, 59) und zwischen diesen an der im Bereich des Steges (54) des Formkörpers (51) liegenden Innenkante (57'') ein weiterer Eingriffnocken (60) für den Eingriff in jeweils drei in einer senkrechten, zur Hülsenlängsachse verlaufenden Ebene liegenden Ausnehmungen (45) der Hülse (30) angeformt sind.

6. Auslaß- und Instrumentenkanal nach einem der Ansprüche 1 bis 5, dadurch gekennzeichnet, daß der Steg (54) des Formkörpers (51) des Reiters (50) federnd-elastisch ausgebildet ist und daß die oberen Schenkelabschnitte (52a, 53a) des Formkörpers (51) die Hülse (30) selbstklemmentd umgreifen.

7. Auslaß- und Instrumentenkanal nach einem der Ansprüche 1-6, dadurch gekennzeichnet, daß die von den beiden oberen Schenkelabschnitten (52a, 53a) begrenzende Kreisfläche einen in etwa dem Außendurchmesser der Hülse (30) entsprechenden Durchmesser aufweist.

8. Auslaß- und Instrumentenkanal nach einem der Ansprüche 1 bis 7, dadurch gekennzeichnet, daß die beiden oberen Schenkelabschnitte (52a, 53a) des Formkörpers (51) des Reiters (50) eine dem Umfang der Hülse (30) entsprechende Formgebung aufweisen.

9. Auslaß- und Instrumentenkanal nach Anspruch 1, dadurch gekennzeichnet, daß der Reiter (250) aus einem etwa U-förmigen Formkörper (251) mit den Hohlzylinder (20) umgreifenden, federnd-elastischen Schenkeln (251a, 251b) besteht, die innenwandseitig ein dem Hohlzylinderprofil entsprechendes Profil aufweisen, wobei der Formkörper (251) zwei mit der Hohlzylinderaufnahmeöffnung (252) des Reiters (250) verbundene Öffnungen (253, 254) zur Aufnahme von Schläuchen, Kabeln od. dgl. aufweist, und daß der Reiter (250) klemmend auf dem Hohlzylinder (20) zur Verhinderung eines Zurückgleitens der Hülse (30) aus der Betriebstellung mit korbartig erweitertem Endumfang der Hülse (30) in die Ausgangsstellung gehalten ist.

10. Auslaß- und Instrumentenkanal nach Anspruch 9, dadurch gekennzeichnet, daß der Hohlzylinder (20) in seiner Außenwandfläch mit einer Ringnut (255) zur Aufnahme der Schenkel (251a, 251b) des Reiters (250) versehen ist.

11. Auslaß- und Instrumentenkanal nach einem der Ansprüche 1 bis 10, dadurch gekennzeichnet, daß der Hohlzylinder (20) im Bereich des spreizbaren Wandabschnittes (35) der Hülse (30) eine Anzahl von über den Zylinderumfang verteilt angeordneten Durchbrechungen (23) aufweist.

12. Auslaß- und Instrumentenkanal nach einem der Ansprüche 1 bis 11, dadurch gekennzeichnet, daß der spreizbare Wandabschnitt (35) der Hülse (30) aus einer Anzahl von über den Hülsenumfang verteilt angeordneten und in Hülsenlängsrichtung verlaufenden, lamellenartigen Formkörpern (135) besteht, die einendseitig (135a) an dem Körper der Hülse (30) und mit ihren anderen Enden (135b) an einem Ringkörper (136) befestigt sind, der mit dem Hohlzylinder (20) fest verbunden ist.

13. Auslaß- und Instrumentenkanal nach einem der Ansprüche 1 bis 11, dadurch gekennzeichnet, daß der spreizbare Wandabschnitt (35) der Hülse (30) von einer Anzahl von über den Umfang verteilt angeordneten und in Hülsenlängsrichtung verlaufenden, in der Hülsenwand (30a) ausgebildeten Schlitzen (236) und den jeweils zwischen den Schlitzen (236) ausgebildeten streifenförmigen Wandabschnitten (235) gebildet ist.

14. Auslaß- und Instrumentenkanal nach einem der Ansprüche 1 bis 13, dadurch gekennzeichnet, daß die lamellenartigen Formkörper (135) und die streifenförmigen Wandabschnitte (235) der Hülse (30) dünnwandig ausgebildet sind und aus einem federn-elastischen Werkstoff bestehen

## Claims

1. Drainage and instruments canal for arthroscopy with a hollow cylinder (20) and a sleeve (30) disposed on the hollow cylinder (20) and displaceable in the longitudinal direction of the hollow cylinder, whose length, in comparison with the length of the hollow cylinder (20), is dimensioned so as to be shorter, in which case the sleeve (30) is provided with a means (40) lockable in position on the wall (20a) of the hollow cylinder (20) and intercating with the hollow cylinder (20) and a wall section (35) which, when the sleeve (30) is longitudinally displaced towards the end (21) of the hollow cylinder (20), can be expanded outwardly, thus enlarging the external diameter of the sleeve in a basket or cage-like fashion, possessing a plurality of perforations (135) disposed so as to be distributed across the circumference and disposed in a side-by-side arrangement,
**characterized in that**
the means (40) for locking the sleeve (30) in position is comprised of an engagement profile (41,45;280) proceeding in the longitudinal direction of the sleeve constructed on the sleeve (30) and a locking or click-locking pin (36,281) disposed within the area of the engagement section (41,45;280) of the sleeve (30) on the external wall (20a) of the hollow cylinder (20) and in that, for positionally securing the drainage and instruments canal (10) in its operative position, the sleeve (30) engaging around the hollow cylinder (20), on its wall (30a), carries a rider (50,250) which can be secured on the same.

2. Drainage and instruments canal according to Claim 1,
**characterized in that**
the means (40) for locking the sleeve (30) in position on the hollow cylinder (20) is comprised of a slot (41) proceeding in the longitudinal direction of the sleeve and running out into the end (32) facing away from the expandable wall section (35) of the sleeve (30) and formed in the sleeve wall possessing a plurality of pitch circle-like recesses or notches (42,142) disposed at regular or irregular intervals in the oppositely located longitudinal rims (41a,41b) of the wall sections (30b,30c) of the sleeve (30) delimiting the slot (41), in which case two each oppositely located recesses or notches (42,142) complement each other in such a way as to form an approximately circular recess or notch (45) and in that, on the outer wall (20a) of the hollow cylinder (20), in the region of the slot (41) of the sleeve (30), an appropriately dimensioned locking pin (36) is disposed and in that, for positionally securing the drainage and instruments canal (10) in its operative position, the sleeve (30) engaging around the hollow cylinder (20), on its wall (30a), carries a rider (50,250) which can be secured on the same.

3. Drainage and instruments canal according to Claim 1,
**characterized in that**
the means (40) for locking the sleeve (30) in position on the hollow cylinder (20), is comprised of a scaly shaped member (280) disposed on the inner wall of the sleeve (30) and of a click-locking pin (281) on the outer wall of the hollow cylinder (20), in which case the scaly section (282) of the shaped member (280) possesses stop edges (283) for the click-locking pin (281) which face the rearward end of the sleeve (30), and in that the click-locking pin (281, for a detachable locking, is swivelable into the wall of the hollow cylinder (20).

4. Discharge and instruments canal according to any of Claims 1 to 3,
**characterized in that,**
for the accommodation of the rider (50), the sleeve (30) of the hollow cylinder (20) in the wall (30a), possesses three rows (R,R1,R2) of consecutively disposed perforations (38) which proceed in the longitudinal direction of the sleeve, in which case, of these three rows (R,R1,R2) with the perforations (38), two rows (R,R1) are formed laterally and the third row (R2) between the two rows (R,R1) and underneath the same in the wall (30a) of the sleeve.

5. Discharge and instruments canal according to any of Claims 1 to 4,
**characterized in that**
the rider (50) is comprised of a shaped member (51) configured in the manner of an "H" possessing pitch circle-like leg sections (52a,53a) located above the web (54) connecting the two longitudinal legs (52,53)) and complementing themselves so as to form an open annulus (55) and with lower leg sections (52b,53b) constructed in the form of handles, in which case, on the internal edges (57,57') facing each other of the two upper leg sections (52a,53a), two oppositely located engaging pins (58,59) and, between the same, on the internal edge (57'') located between them within the region of the of the web (54) of the shaped member (51), a further engaging pin (60) for engagement into, in each case, three recesses or notches (45) of the sleeve (30) in a vertical plane proceeding relative to the longitudinal axis of the sleeve, are formed on.

6. Discharge and instruments canal according to any of Claims 1 to 5,
**characterized in that**
the web (54) of the shaped member (51) of the rider (50) is constructed so as to be springably elastic and in that the upper leg sections (52a,53a) of the shaped member (51) engage around the sleeve (30) in a self-clamping function.

7. Discharge and instruments canal according to any of Claims 1 to 6,
**characterized in that**
the circular area delimited by the two upper leg sections (52a,53a) possesses a diameter which corresponds approximately to the external diameter of the sleeve.

8. Discharge and instruments canal according to any of Claims 1 to 7,
characterized in that
the two upper leg sections (52a,53a) of the shaped member (51) of the rider (50) possess a configuration corresponding to the circumference of the sleeve (30).

9. Discharge and instruments canal according to Claim 1,
**characterized in that**
the rider (250) is comprised of a shaped member configured approximately like a "U" (251) with springably elastic legs (251a,251b) engaging around the hollow cylinder (20) which, on the side of the inner wall, possess a profile corresponding to the profile of the hollow cylinder (20), in which case the shaped member (251) possesses two apertures (253,254) connected with the receiving aperture (252) of the hollow cylinder of the rider (250) for the accommodation of tubes, cables or suchlike, and in that the rider (250) is clampingly retained on the hollow cylinder (20) so as to prevent a sliding back of the sleeve (30) from the operative position with a basket or cage-like expanded terminal circumference of the sleeve (30) into the initial position.

10. Discharge and instruments canal according to Claim 9,
**characterized in that**
the hollow cylinder (20), in its outer wall area, is provided with an annular groove (255) for the accommodation of the legs (251a,251b) of the rider (250).

11. Discharge and instruments canal according to any of Claims 1 to 10,
**characterized in that**
the hollow cylinder (20), within the area of the expandable wall section (35) of the sleeve (30), possesses a plurality of perforations (23) disposed so as to be distributed over the cylinder circumference.

12. Discharge and instruments canal according to any of Claims 1 to 11,
**characterized in that**
the expandable wall section (35) of the sleeve (30) is comprised of a plurality of lamellar members (135) disposed so as to be distributed over the sleeve circumference and proceeding in the longitudinal direction of the sleeve, which are, at one end (135a), attached to the body of the sleeve (30) and, with their other ends (135b), to an annular member (136) which is rigidly connected to the hollow cylinder (20).

13. Discharge and instruments canal according to any of Claims 1 to 11,
**characterized in that**
the expandable wall section (35) of the sleeve (30) is constituted of a plurality of slots (236) disposed so as to be distributed over the cirumference and proceeding in the longitudinal direction of the sleeve, constructed in the sleeve wall (30a) and the, in each case, strip-like wall sections (235) constructed between the slots (236).

14. Discharge and instruments canal according to any of Claims 1 to 13,
**characterized in that**
the lamellar members (135) and the strip-like wall sections (235) of the sleeve (30) are constructed so as to be thin-walled and are comprised of a springably elastic material.

## Revendications

1. Canal d'instruments et de drainage pour l'arthroscopie avec un cylindre creux (20) et une douille (30), placée sur le cylindre creux (20) et translatable dans le sens longitudinal du cylindre creux, douille dont la longueur est plus courte que la longueur du cylindre creux (20), la douille (30) étant pourvue d'un dispositif (40) pouvant être bloqué sur la paroi (20a) du cylindre creux (20), coopérant avec le cylindre creux (20) et présentant une portion de paroi (35), qui, lors d'une translation longitudinale de la douille (30) vers l'extrémité (21) du cylindre creux (20), peut s'écarter vers l'extérieur et agrandit le diamètre extérieur de la douille en panier, avec un nombre de découpures (135) juxtaposées placées en étant réparties sur la périphérie, **caractérisé en ce** que le dispositif (40) pour bloquer la douille (30) sur le cylindre creux (20) est constitué par un profil d'engrènement (41, 45 ; 280) configuré sur la douille (30) dans le sens longitudinal de la douille et par un ergot de blocage et d'enclenchement (36, 281) dans la zone du profil d'engrènement (41, 45 ; 280) de la douille (30) placé sur la paroi extérieure (20a) du cylindre creux (20) et que, pour bloquer la position du canal d'instruments et de drainage (10) dans sa position d'utilisation, la douille (30) qui entoure le cylindre creux (20) porte sur sa paroi (30a) un curseur (50, 250) qui peut être bloqué sur celle-ci.

2. Canal d'instruments et de drainage selon la revendication 1, **caractérisé en ce** que le dispositif (40) pour bloquer la douille (30) sur le cylindre creux (20) est constitué par une fente (41) qui va dans le sens longitudinal de la douille, qui est configurée dans la paroi de la douille et qui se termine dans l'extrémité (32) détournée de la portion de paroi (35) de la douille (30) qui peut s'écarter, fente avec un certain nombre d'évidements en forme de cercle partiel (42, 142) placés à des intervalles égaux ou inégaux dans les bords longitudinaux (41a, 41b) opposés des portions de paroi (30b, 30c) de la douille (30) qui délimitent la fente (41), deux évidements (42, 142) situés respectivement l'un en face de l'autre se complétant respectivement en un évidement à peu près circulaire (45) et qu'un ergot d'arrêt (36), dimensionné avec un diamètre correspondant à peu près à celui de l'évidement (45), est placé sur la paroi extérieure (20a) du cylindre creux (20) dans la zone de la fente (41) de la douille (30) et que, pour bloquer la position du canal d'instruments et de drainage (10) dans sa position d'utilisation la douille (30) qui entoure le cylindre creux (20) porte, sur sa paroi (30a), un curseur (50, 250) qui peut être bloqué sur celle-ci.

3. Canal d'instruments et de drainage selon la revendication 1, **caractérisé en ce** que le dispositif (40) pour bloquer la douille (30) sur le cylindre creux (20) est constitué par un corps profilé (280) de type écaille placé sur la paroi intérieure de la douille (30) et par un ergot d'enclenchement (281) sur la paroi extérieure du cylindre creux (20), le profilé en écaille (282) du corps profilé (280) présentant des bords de butée (283) pour l'ergot d'enclenchment (281) qui sont tournés vers l'extrémité arrière de la douille (30) et que l'ergot d'enclenchement (281) peut être pivoté dans la paroi du cylindre creux (20) pour un desserrage du blocage.

4. Canal d'instruments et de drainage selon l'une des revendications 1 à 3, **caractérisé en ce** que, pour loger le curseur (50), la douille (30) du cylindre creux (20) présente dans la paroi (30a) trois rangées (R, R1, R2) dans le sens longitudinal de la douille de découpures (38) placées les unes derrière les autres, deux rangées (R, R1) parmi ces trois rangées (R, R1, R2) avec les découpures (38) étant configurées sur le côté et la troisième rangée (R2) étant configurée entre les deux rangées (R, R1) et au-dessous de celles-ci dans la paroi (30a) de la douille (30).

5. Canal d'instruments et de drainage selon l'une des revendications 1 à 4, **caractérisé en ce** que le curseur (50) est constitué par un corps moulé en forme de H (51) avec des portions de montants en forme de cercle partiel (521, 53a) qui se situent au-dessus de la barrette (54) qui relie les deux montants longitudinaux (52. 53), qui se complètent en un anneau ouvert (55) et avec des portions de montants inférieures (52b, 53b) configurées comme des manettes, deux ergots d'engrènement (58, 59) opposés l'un à l'autre étant formés sur les bords intérieurs (57, 57') tournés l'un vers l'autre des deux portions supérieures de montants (52a, 53a) et un autre ergot d'engrènement (60) pour l'engrènement étant formé entre ces deux ergots sur le bord intérieur (57'') qui se situe dans la zone de la barrette (54) du corps moulé (51), ces ergots étant moulés dans respectivement trois évidements (45) de la douille (30) qui se situent dans un plan perpendiculaire à l'axe longitudinal de la douille.

6. Canal d'instruments et de drainage selon l'une des revendications 1 à 5, **caractérisé en ce** que la barrette (54) du corps moulé (51) du curseur (50) est configurée élastique à la manière d'un ressort et que les portions supérieures de montant (52a, 53a) du corps moulé (51) enveloppent la douille (30) par autoserrage.

7. Canal d'instruments et de drainage selon l'une des revendications 1 à 6, **caractérisé en ce** que la surface circulaire qui est délimitée par les deux portions de montant supérieures (52a, 53a) présente un diamètre qui correspond à peu près au diamètre extérieur de la douille (30).

8. Canal d'instruments et de drainage selon l'une des revendications 1 à 7, **caractérisé en ce** que les deux portions supérieures de montant (52a, 53a) du corps moulé (51) du curseur (50) présentent une configuration qui correspond à la périphérie de la douille (30).

9. Canal d'instruments et de drainage selon la revendication 1, **caractérisé en ce** que le curseur (250) est constitué par un corps moulé (251) à peu près en forme d'U (251) avec des montants élastiques à la manière d'un ressort (251a, 251b) qui enveloppent le cylindre creux (20) qui présentent, sur le côté de la paroi intérieure, un profil qui correspond au profil du cylindre creux, le corps moulé (251) présentant deux orifices (253, 254) pour le logement de tuyaux, câbles ou équivalent qui sont reliés à l'orifice de logement du cylindre creux (252) du curseur (250) et que le curseur (250) est maintenu par coïncement sur le cylindre creux (20) pour éviter que la douille (30) ne revienne par glissement de la position d'utilisation avec la périphérie d'extrémité de la douille (30) élargie en panier à la position de départ.

10. Canal d'instruments et de drainage selon la revendication 9, **caractérisé en ce** que le cylindre creux (20) est pourvu dans sa surface de paroi extérieure d'une gorge annulaire (255) pour loger les montants (251a, 251b) du curseur (250).

11. Canal d'instruments et de drainage selon l'une des revendications 1 à 10, **caractérisé en ce** que le cylindre creux (20) présente dans la zone de la portion de paroi (35) de la douille (30) qui peut être écartée un certain nombre de découpures (23) placées en étant réparties sur la périphérie du cylindre.

12. Canal d'instruments et de drainage selon l'une des revendications 1 à 11, **caractérisé en ce** que la portion de paroi (35) de la douille (30) qui peut être écartée est constituée par un certain nombre de corps moulés (135) placés en étant répartis sur la périphérie de la douille, en type de lamelle dans le sens longitudinal de la douille qui sont fixés à l'une des extrémités (135a) au corps de la douille (30) et avec leurs autres extrémités (135b) à un corps annulaire (136) qui est relié de manière fixe au cylindre creux (20).

13. Canal d'instruments et de drainage selon l'une des revendications 1 à 11, **caractérisé en ce** que la portion de paroi (35) qui peut être écartée de la douille (30) est formée par un certain nombre de fentes (236) placées en étant réparties sur la périphérie et configurées dans la paroi de la douille (30a) en allant dans le sens longitudinal de la douillle et par les portions de paroi (235) en forme de bandes formées entre les fentes (236).

14. Canal d'instruments et de drainage selon l'une des revendications 1 à 13, **caractérisé en ce** que les corps moulés de type lamelle (135) et les portions de paroi en forme de bande (235) de la douille (30) sont configurés avec des parois minces et sont constitués par une matière élastique à la manière d'un élastique.
